(19) Europäisches Patentamt / European Patent Office / Office européen des brevets

(11) **EP 3 907 741 A1**

(12) **EUROPEAN PATENT APPLICATION**

(43) Date of publication:
**10.11.2021 Bulletin 2021/45**

(51) Int Cl.:
**G16H 50/80** (2018.01)    **H04W 4/00** (2018.01)
**G08B 21/00** (2006.01)

(21) Application number: **20173770.7**

(22) Date of filing: **08.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universität Wien**
**1010 Wien (AT)**

(72) Inventors:
• HENZINGER, Monika
  1090 Vienna (AT)
• NOE, Alexander
  1090 Vienna (AT)
• GANSTERER, Wilfried
  1090 Vienna (AT)

(74) Representative: **Karl, Christof**
**Bardehle Pagenberg Partnerschaft mbB**
**Patentanwälte, Rechtsanwälte**
**Prinzregentenplatz 7**
**81675 München (DE)**

(54)    **WEIGHTED INFECTION ALERT SYSTEM BETWEEN PORTABLE DEVICES**

(57)    The present invention provides a computer-implemented method for determining the likelihood of infection of a user of a portable electronic device by enabling assigning to each user (of a portable electronic device with an infection tracking app) a value corresponding to an infection risk score wherein, the higher the score, the higher the risk of infection, wherein a recursive ranking procedure is performed by the portable device of the user and/or a server communicating with the portable device of the user, in which a change in the score of a user means that the scores of their "contacts" can proportionally change as well, which in turn can change the values of the "contacts" of the "contacts" of the user.

FIG. 2

EP 3 907 741 A1

## Description

### Technical field

The present invention relates to a computer-implemented method for determining the likelihood of infection of a user of a portable electronic device.

### Background

Current computer software applications i.e. smartphone apps for managing the spread of infections inform their user only whether they were in close proximity contact with an infected person within a given time frame or not. In other words, conventional techniques effectively involve a binary indication of whether a person is infected or not.

In contrast to apps such as "Stopp Corona" which provide data on whether a user of the app is infected or not, it would be desirable to rather provide data on how likely a user who is not yet established as being infected or not, to be infected based on their "contacts" i.e. data of other people (also users of the app) with which the user has had close proximity contact with. A user who has had close proximity contact with many infected users is more likely to be infected than a user who has had no contact with infected people. Therefore, the former also has a higher risk of infection.

The present invention aims to provide a new approach by enabling assigning to each user (of a portable electronic device with an infection app such as the aforementioned) a value corresponding to an infection risk score wherein, the higher the score, the higher the risk of infection. The invention proposes to solve the aforementioned problem by providing a computer-implemented method wherein a recursive ranking procedure is performed by the portable device of the user and/or a server communicating with the portable device of the user, in which a change in the score of a user means that the scores of their "contacts" can proportionally change as well, which in turn can change the values of the "contacts" of the "contacts" of the user, etc.

### Summary

The present invention is defined by the features of the independent claims. Preferred embodiments thereof are defined by the sub-features of the dependent claims. The computer-implemented method of the present invention may be performed by one or more portable electronic devices such as smartphones or wearables such as smart watches and/or as part of a wireless network including one or more servers.

There are three main aspects according to the invention which can be used alone or in combination.

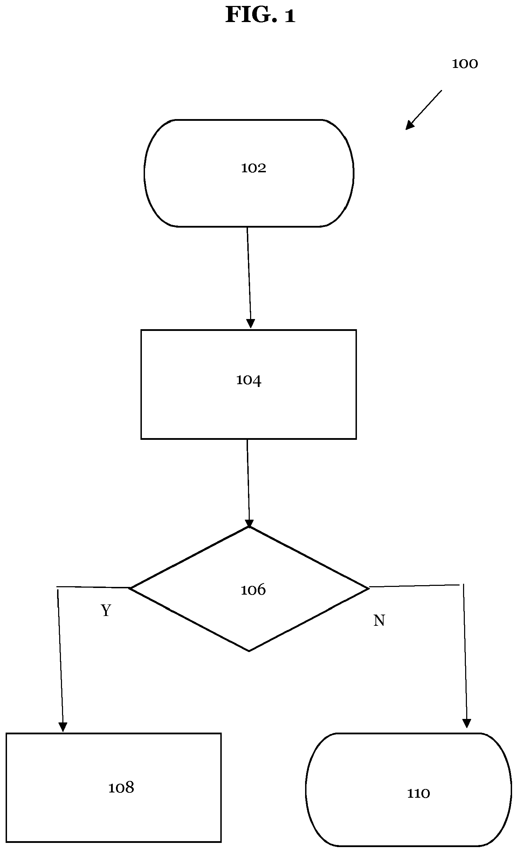

According to a first aspect of the invention shown in Fig. 1, a computer-implemented method 100 is provided comprising receiving 102, at a first portable electronic device of a first user, a first notification including a first value representing a level of risk of infection associated with a second user of a second portable electronic device which has been determined to have been in close proximity to the first portable electronic device. Subsequently, a first score representing a level of risk of infection associated with the first user is updated 104 based on the received value. Thereafter, a determination 106 is performed as to if the updated score is greater or less than a predetermined score. If it is determined that the updated score is greater than the predetermined score 108, a second notification including the second value is sent to at least one third portable electronic device associated with a third user which has been determined to have been in close proximity to the first portable electronic device for updating a second score representing a level of risk of infection associated with the third user. The sending of the second notification may either be automatic, or in response to the reaction of the first user to a prompt displayed on the first portable electronic device. If it is determined that the updated score is lower than the predetermined score 110, then no further action is taken.

According a preferred example of the first aspect, the values and scores are numerical values between 0 and 1. Recursive computation of a risk score may be applied wherein, for example, each infected user gets a score of 1 and the score of a not-known-to-be-infected user A depends on a function (e.g. a fraction of the total score) of the risk score of the users with which A has been in close proximity contact with within a given time frame. This advantageously enables not only binary indication of infection, but also fractional values of infection risk of a user i.e. indicating the likelihood of infection of each user.

An advantage of this approach according to the present invention is that it takes into account the nature of a highly contagious infection such as Covid-19 wherein the generation time is shorter than the incubation time and also takes into consideration the infection of (still) asymptomatic patients. Implementing quarantine of all contacts of all contacts of infected users is very high impact on healthcare resources. Therefore, according to the approach of the present invention, only people who are determined to have had contact with people who have a high chance of infection are warned. Since the method according to the present invention is recursive, it also enables longer infection paths to be mapped. By including a user query, the privacy of the user can be protected i.e. the infection score remains local to the device and is only sent if the user agrees.

Optionally, the first notification is received from a server and/or the second notification is sent from a server. This enables the server to provide centralised storage of infection risk information of all users with portable electronic devices, thereby improving the accuracy of the overall data.

Alternatively, the first notification is received directly from the second portable electronic device and/or the second notification is sent from the first portable electronic device. This enables direct peer-to-peer communication of infection risk, thereby improving real-time notification by obviating the need for further links via a server. This requires that phones exchange their contacts (i.e. telephone numbers) directly.

In another preferred example of the first aspect or examples thereof, one of either of the notifications may be received from a server and the other of either of the notifications maybe received from one of the portable electronic devices. This enables improvement of both the accuracy and timely reception of the infection risk data depending on the circumstances.

In further preferred example of the first aspect or above examples thereof, the second portable electronic device is determined to have been in close proximity to the first portable electronic device by means of exchanging identification data unique to the devices. Preferably, exchanging identification data comprises, by at least one of the first and second portable electronic devices, periodically generating a unique random number and wirelessly sending the unique random number to the other of the first and second portable electronic devices whenever it is determined that the first and second portable electronic devices are in close proximity to one another, and storing the received unique random number on the first or second portable electronic device respectively. Furthermore, the method also preferably involves, by the first portable electronic device, periodically sending a request to a server to determine if a notification associated with the unique random number of the first portable electronic device is stored, wherein the notification corresponds to the first notification. Moreover, the method also preferably further comprises, if it is determined that the updated score is greater than the predetermined score, sending, to the server, the updated score and a list of the unique random numbers of all portable electronic devices that the first portable electronic device has been determined to have been in close proximity to, and, by the server, storing for each received unique random number a score based on the updated score of the first portable electronic device so that whenever a portable electronic device of all portable electronic devices that the first portable electronic device has been determined to have been in close proximity to sends a notification associated with its unique random number to the server, the server will send the updated score of the first portable electronic device.

According to a further preferred example of the first aspect or examples thereof, data including a number of times and/or the total duration the second portable device is determined to have been in close proximity to the first portable electronic device is stored in a memory of the first portable electronic device and/or the second portable electronic device. This enables a device to improve accuracy of infection risk data by further increasing the infection score of a user if contact with an infected person is frequent and/or prolonged.

According to a further preferred example of the first aspect or aforementioned examples thereof, if it is determined that the updated score is greater than the predetermined score, an alert notification is displayed to the user on the first portable electronic device. The alert notification may be displayed to the user by means of at least one of text, email, changing the contrast, colour and/or appearance of the app background or smartphone background. This prompts the user to go to get tested or implement self-quarantine.

According to a further preferred example of the first aspect or aforementioned examples thereof, the method further comprises determining a location and time for each of the determinations of the portable electronic devices having been in close proximity to one another and displaying the determined location and times on a map on the first portable electronic device. This advantageously offers convenient overview of places and times where contact with high-risk individuals transpired, thereby allowing the user to avoid these places and times to reduce the risk of infection.

According to a further preferred example of the first aspect or aforementioned examples thereof, if it is determined that the updated score is greater than the predetermined score, an alert notification is sent by the first portable electronic device to a server of an organisation managing the spread of infection. These messages enable a global organisation such as the World Health Organisation or any domestic health department of a state to compile accurate statistics in real time.

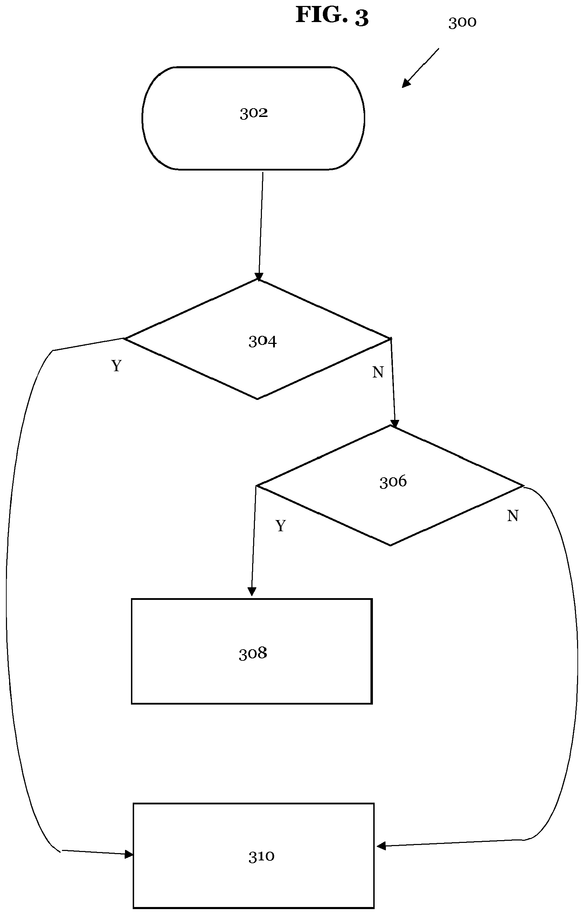

According to a second aspect of the present invention shown in Fig. 3, a computer-implemented method 300 is provided comprising receiving 302, at an electronic device at a first time T1, a first notification that a first user of a first portable electronic device is infected. In response to the first notification, one or more identifiers of at least one second user of at least one second portable electronic device determined to have been in close proximity with the first portable electronic device are stored in a list 310. Subsequently, a second notification that a third user of a third portable electronic device is infected is received at the electronic device at a second time T2 after the first time. In response to the second notification, it is determined whether the third portable electronic device has been in close proximity with the first portable electronic device and the second portable electronic device after the second portable electronic device was determined to have been in close proximity with the first portable electronic device. If it is determined 306 that the third portable electronic device has been in close proximity with the second portable electronic device but not with the first portable electronic device and it is determined 304, 306 that there are no further portable electronic devices that have been determined to have been in close proximity of the third portable electronic device, the electronic device sends 308 an alert notification to the second portable electronic device.

In a preferred example of the second aspect of the invention, if it is determined that the third portable electronic device has been in close proximity with the second portable electronic device but not with the first portable electronic device and it is determined 304, 306 that there are no further portable electronic devices that have been determined to have been in close proximity of the third portable electronic device, the electronic device sends 308 an alert notification to one or more portable electronic devices of one or more users identified in the list which have been determined to have been in close proximity to the second portable electronic device.

Preferably, the alert notification results in performing the aforementioned method steps of the first aspect of the invention or examples thereof.

Furthermore, preferably the electronic device is a server and/or at least part of the list is stored 310 on the electronic device and/or one or more of the portable electronic devices. By storing at least part of the list on the one or more of the portable electronic devices this thereby improves the ability to anonymise user data and improve privacy due to the decentralisation i.e. the data can be distributed throughout a peer-to-peer network of the portable electronic devices of the users. Alternatively, the different (partial) lists from all portable electronic devices may also be centralised in a global database which could be stored on a single server.

According to the second aspect of the invention, a determination of infection carriers is performed by, for example, assuming that an infected user A has contact to user B at time T1 and user B has contact to user C at time T2 with T1 < T2, it is determined that B has an infection path of length 1 and C is determined to have an infection path of length 2. If it is subsequently determined that user C is infected, but C did not have any other infection path of length 1 or 2, the invention detects such a setting, even in a distributed system, and assigns to user B a high-risk score.

According to a third aspect of the invention which may be combined with the first and/or second aspects of the invention, decentralised and asynchronous approximation of the infection risk scores of users is performed. Based on distributed variants of sparse matrix-vector multiplication, the risk scores are approximated without collecting contact information centrally, thereby reducing the amount of synchronisation of the participating apps needed.

**Detailed description of implementations**

According to the prior art, it is known to provide a list of neighbours (each with their own unique ID and time) i.e. users which have been determined by their portable electronic devices such as smartphones, wearables or the like, to have had contact with one another by virtue of having been in close proximity to one another. Therefore, these determined neighbours maybe referred to as "contacts" which, in the context of the present invention, does not necessarily refer to the contact details of individuals stored locally on the portable electronic device such as telephone numbers and e-mail addresses, but rather persons i.e. users of other portable electronic devices who have been determined to have been in close proximity to one another e.g. by means of receipt or exchange of identification data via a wireless signal such as Bluetooth or Wi-Fi and may be stored locally within the infection management app installed on the portable electronic device. A given user's infection score i.e. the binary indication of the probability that the user is infected with e.g. Covid-19 is initially zero. For example, if the user N is infected, the infection score of N is set locally in the portable electronic device to and a warning is sent to contacts (in the case of the "Stopp Corona" app). This warning includes infection score INS (i.e. for N).

Recursive computation of a risk score

However, according to the novel approach of the present invention, in response to such a warning of infection, each contact K of N may increase its own local infection score by a factor of the infection score between 0 and 1 (e.g. ~0.1 * INS). If a user K exceeds their own infection threshold (e.g. 0.3 to 0.5), a notification and prompt such as "The chance that you are infected has exceeded a certain threshold. Would you like to inform your contacts anonymously?" may be displayed on the portable electronic device. If the user selects "yes", the device may send an infection message with their local infection score to their contacts, thereby increasing the respective infection scores of the contacts by a factor of the received score. Alternatively, the notification maybe sent automatically to the contacts via a server and/or directly. Thus, the chance of infection based on a person who is infected with 50% probability is 50% as high as the chance of infection based on a person confirmed as infected. For each contact if it is determined that their increased infection scores exceed a threshold, further messages may be sent to their neighbours.

The infection score of a user N can therefore be described as follows:

$$INS\_N = \text{sum of all K in Kt(N) } INS\_K * gamma$$

wherein INS_K is the infection score of contact K and Kt(N) is the list of all contacts of N who have published their infection score.

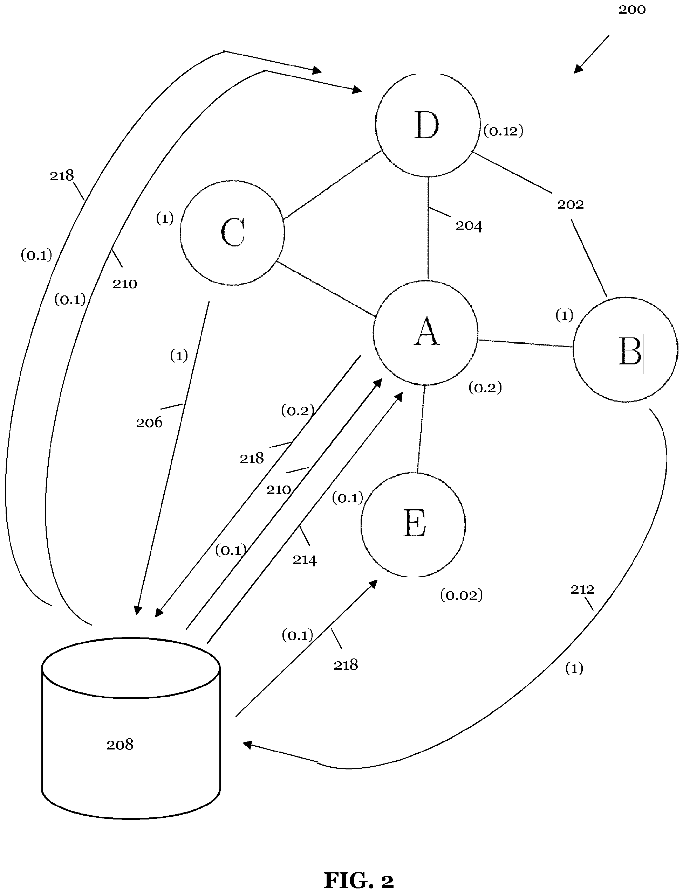

According to a preferred example 200 of the first apect shown in Fig. 2, A, B, C, D, E respectively correspond to the unique IDs of users 202. Connections 204 between users are determined by virtue of one or more of the users 202 having had close proximity contact with one another as determined above according to the respective portable electronic devices of the users detecting close proximity to one another e.g. via wireless exchange of unique IDs of their devices as described in more detail above in the summary and below with respect to a preferred implementation.

According to this preferred example, user C is determined to be infected and sends 206, from C's smartphone infection score (1) to a server 208 along with a list of the most recent contacts of C which have been in close proximity to C i.e. C's smartphone. The server 208 then sends a notification 210 including a value representing a level of risk of infection associated with C to the smartphones of users A and D i.e. contacts of C which have been determined, either by the server 208, or C's smartphone, to have been in close proximity to C i.e. C's smartphone. In response to receiving the notification, the respective infection scores of users A and D are updated on their smartphones as follows:

$$\text{INS\_A} = 0 \, (\text{before}) + 0.1 \, (\text{gamma}) * 1 \, (\text{INS\_C}) = 0.1$$

$$\text{INS\_D} = 0 \, (\text{before}) + 0.1 \, (\text{gamma}) * 1 \, (\text{INC\_C}) = 0.1$$

wherein the following exemplary values are used: Factor gamma = 0.1, threshold for infection warning = 0.15.

User B is determined to be infected and sends 212, from B's smartphone, an infection score (1) to the server 208 along with a list of the most recent contacts of B which have been in close proximity to B i.e. B's smartphone. The server 208 then sends a notification 214 including a value representing a level of risk of infection associated with B to the smartphone of user A i.e. a contact of B which has been determined, either by the server 208, or B's smartphone, to have been in close proximity to B i.e. B's smartphone. User A receives the notification on their smartphone and the infection score of user A is updated as follows:

$$\text{INS\_A} = 0.1 \, (\text{before}) + 0.1 \, (\text{gamma}) * 1 \, (\text{INS\_B}) = 0.2$$

In response to the infection score of A having been updated, A's smartphone then determines if the updated score is greater or less than a predetermined (threshold) score i.e. 0.15. Once it is determined that A's infection score exceeds the predetermined threshold of 0.15, A's smartphone sends 216 the updated infection score (0.2) to the server along with a list of the most recent contacts of A which have been in close proximity to A i.e. A's smartphone.

In response to the server having received the updated infection score (0.2) from A's smartphone, the server sends respective notifications to the respective smartphones of users D and E i.e. contacts of A which have been determined, either by the server, or A's smartphone, to have been in close proximity to A i.e. A's smartphone. In response to receiving the notification, the respective infection scores of D and E are updated on their smartphones as follows:

$$\text{INS\_D} = 0.1 \, (\text{before}) + 0.1 \, (\text{gamma}) * 0.2 \, (\text{INS\_A}) = 0.12$$

$$\text{INS\_E} = 0 \, (\text{before}) + 0.1 \, (\text{gamma}) * 0.2 \, (\text{INS\_A}) = 0.02$$

Since users B and C have already been determined to be infected, no further action is taken.

According to a preferred implementation of the aforementioned preferred example, any reference to sending a list of most recent contacts includes sending a list of the unique random number of the most recent contacts. Furthermore, any reference to the server sending a notification to the smartphone includes a server storing, for every random number it ever received all the infection scores of smartphones that have been in close contact with a smartphone with this unique random number. Moreover, according to this preferred implementation, every smartphone frequently sends to the server its unique random number and asks whether there are infection score(s) stored for this unique random number. If so, the server sends the infection score(s) stored for its random number to the smartphone in response.

Thus, in a preferred example of the aforementioned preferred implementation, every few days (or some not so frequent time interval) each portable electronic device generates a unique random number and whenever it is in close contact i.e. proximity to another portable electronic device, it wirelessly sends this random number to the other portable electronic device. The other portable electronic device stores this random number. The first portable electronic device receives the random number of the other portable electronic device. Very frequently (for example every 30 minutes) each portable electronic device checks with the server asking whether it has a message for its random number. If there is a message this corresponds to the first notification in the aforementioned first aspect of the invention. The portable electronic device then updates its score and if the score is above a given threshold it sends this new score together with a list of the random numbers of all portable electronic devices that it has been in close contact with to the server. The server stores for each such random number these scores and whenever a portable electronic device with one of these random numbers checks in with the server, the server sends it the new score of the first portable electronic device. The advantage of this scheme is that the server never has to store a risk score R together with the phone number or the random number of that portable electronic device. It only stores the risk score of the random numbers that were in close contact with the person with this risk score R. This is how it is envisaged that a new app will implement the information exchange between portable electronic devices.

Therefore, in a preferred implementation of the first aspect of the present invention, a user X will be informed in the "Stopp Corona" app if one of their contacts is infected with COVID-19. This increases the probability that X is infected as well, because they could have been infected by that contact. X can locally calculate a value based on the probability of infection from contact with infected persons, which should estimate how likely it is that X is also infected. If this value exceeds a pre-defined threshold, X should inform all their recent contacts so that they too can update their infection score. A big advantage of this variant compared to the current version is that it also models the risk of infection by pre- or asymptomatic users who do not (yet) know about their infection, and people who have had a lot of contact with potentially infected people can also be warned. The privacy policy of the "Stopp Corona" app currently only includes the sending of warnings in case of confirmed infection. This could possibly be supplemented by the fact that even if a threshold value ("possibly infected") is reached, the app will inform all contacts so that when the threshold value is reached, their app will inform their users. This value can also be used to find test candidates, because people with a high value should preferably be tested for an infection, for example with COVID-19. So one could inform users with a high value that they could be tested for infection with, for example SARS-COV-2 viruses.

Determination of infection carriers

In a preferred implementation of the second aspect of the present invention, since a relatively high proportion of infected people are asymptomatic, but can nevertheless be infectious such as in that case of people infected with COVID-19 or SARS-COV-2, it is important to have asymptomatically infected persons tested. According to an example, person A is a person who reports being infected and has had contact with a person B at the time T1. This is determined to be a contact path of length 1. Furthermore, person C is a person who later reports being infected and currently at T2 has had contact with person B, wherein T1 is before T2. This is determined to be a contact path of length 2.

The server can now determine that person B had contact with several people who are known to be infected with COVID-19. If person C has had no other contact with infected persons and has no other infected persons with a contact path of length 2, it is likely that person B is the carrier of the virus from person A to person C and has an asymptomatic course of the disease. To avoid further infection, person B can now be informed by activating an alert in their smartphone that they may be infected and could be tested for, for example, SARS-COV-2.

According to the privacy policy of the "Stopp Corona" app, the app of each newly infected person sends his Unique Identifier and also the Unique Identifiers of their contacts of the last 54 hours to a server. This information corresponds to the contact paths of the infected person of length 1. Assuming that this information is stored on the server for several days, the server checks for each newly infected person whether his contacts are already in the contact list of another infected person (i.e. the server calculates the contact paths of length 2). The server can then store how many contact paths of length 2 it has for each Unique Identifier.

If the same Unique Identifier is now found in the contact list of at least two infected persons, the server can check whether the Unique Identifier fulfils the above conditions (of person B), i.e. if they had contact with an infected person A at time T1 and had contact with another infected person C at a later time T2 and C has no other contact paths of length 1 or 2, then person B is a possible carrier.

Since the app only sends the contacts of infected users according to the privacy policy, contact paths with more than one intermediate step, i.e. with a length greater than 2, are not traceable. Currently, only contacts of the last 54 hours before the infection is reported are transmitted to the server. However, due to the long incubation period of COVID-19, contact with the person from whom the infected person was infected is probably further in the past. To find the carrier, the time of the transmitted contacts might have to be increased.

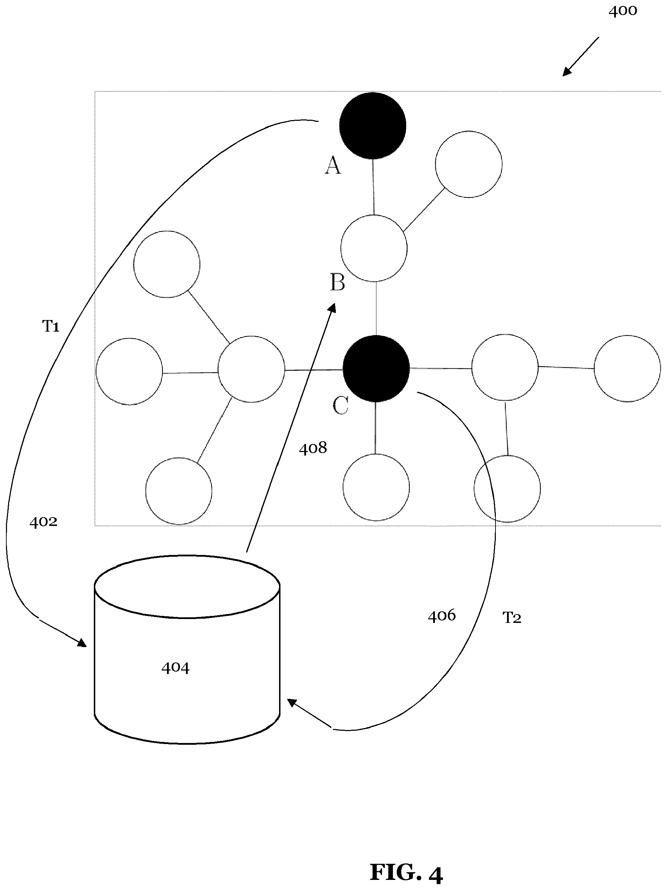

In a preferred implementation 400 of the second aspect of the invention shown in Fig. 4, user A of a smartphone is determined to be infected at a first time T1, and user A's smartphone sends 402 a notification to a server 404 indicating that user A is infected. In response to the notification that user A is infected, one or more identifiers of one or more contacts of A i.e. users of smartphones determined to have been in close proximity with user A's smartphone may be stored in a list at least partially stored on the server (e.g. as part of a global database) such as user B. User C, who is not a direct contact of user A (i.e. user C's smartphone has not been in close proximity to user A's smartphone), but is a direct contact of user B (i.e. user C's smartphone has been in close proximity with user B's smartphone) is subsequently determined to be infected at a second time T2 and a notification is sent 406 from user C's smartphone to the server indicating that user C is infected. Although it is determined by user C's smartphone that user C has no direct contacts who are infected, in response to the notification that user C is infected, the server determines that user B's smartphone has been in close proximity with user A's smartphone. Therefore, since user A is determined to be the only infected user that has a path of length 2 to C, there is a high likelihood that the infection passed from user A to user C via user B, wherein the infection of user B remained undetected. In response to the server determining that user B's smartphone has been in close proximity with user A's smartphone, an alert notification is sent 408 to user B's smartphone (and possibly also to a further server such as one belonging to a health organisation) indicating that user B is likely infected in order to prompt user B to be tested for infection.

In other words, if the server knows all contacts of infected persons, the server can determine and warn user B. If not, user C must additionally send a notification to the server that it has no known infected contacts. This information is then seen by C's neighbours and they can check if they have any other infected contacts (like user A in the example of user C). User B would then have to send this information to the servers. If the server receives only one such message, there is only one contact in two degrees of freedom that is confirmed to be infected, and a likely path of infection has been determined.

As in the case of the preferred implementation of the preferred example of the first aspect, any reference to sending a list of most recent contacts includes sending a list of the unique random number of the most recent contacts. Furthermore, any reference to the server sending a notification to the smartphone includes a server storing, for every random number it ever received all the infection scores of smartphones that have been in close contact with a smartphone with this unique random number. Moreover, according to this preferred implementation, every smartphone frequently sends to the server its unique random number and asks whether there are infection score(s) stored for this unique random number. If so, the server sends the infection score(s) stored for its random number to the smartphone in response.

Furthermore, preferably at least part of the list is stored on one or more of the portable electronic devices of users A, B, and/or C. As alluded to in the summary, by storing at least part of the list on the one or more of the portable electronic devices this thereby improves the ability to anonymise user data and improve privacy due to the decentralisation i.e. the data can be distributed throughout a peer-to-peer network of the portable electronic devices of the users. The advantage of this approach is that it enables protection of data of the users whilst automatically allowing the tracking of infection paths.

Decentralisation

If the model is suitable, the first aspect of the invention can be created by repeated multiplication of the "contact matrix" (the matrix that contains the graph of all contacts of all app users) with a (e.g. randomly selected) start vector can be implemented algorithmically. Depending on the selected distribution of the data required for this purpose, different degrees of decentralisation, reduction of the synchronisation effort as well as different possibilities of updating (feeding new information about detected infections) are possible. In this way, every user of the app can continuously update his own infection score by communicating with his contacts (either passively, e.g. if one of his contacts informs about a detected infection; or actively, e.g. if he recently had new contacts).

On the basis of suitable indexing (order in the unique IDs for users of the app) and corresponding conceptual distribution of the "contact matrix", different variants of distributed matrix-vector multiplication can be used to perform the estimation of infection scores in a decentralised manner and with as little synchronisation effort as possible. Since in the concrete situation a modelling with undirected graphs is sufficient, further simplifications and efficiency increases are possible.

**Claims**

1.  A computer-implemented method (100) comprising the steps of:

> receiving (102), at a first portable electronic device of a first user, a first notification including a first value representing a level of risk of infection associated with a second user of a second portable electronic device which has been determined to have been in close proximity to the first portable electronic device;
> updating (104) a first score representing a level of risk of infection associated with the first user based on the received first value;
> determining (106) if the updated score is greater or less than a predetermined score; and
> if it is determined that the updated score is greater than the predetermined score, sending (108) a second notification including the second value to at least one third portable electronic device associated with a third user which has been determined to have been in close proximity to the first portable electronic device for updating a second score representing a level of risk of infection associated with the third user.

2. The method of claim 1, wherein the values and scores are numerical values between o and 1.

3. The method of claims 1 or 2, wherein the first notification is received from a server and/or the second notification is sent from a server.

4. The method of claims 1 or 2, wherein the first notification is received directly from the second portable electronic device and/or the second notification is sent from the first portable electronic device.

5. The method of any of the preceding claims, wherein the second portable electronic device is determined to have been in close proximity to the first portable electronic device by means of exchanging identification data unique to the devices.

6. The method of claim 5, wherein exchanging identification data comprises, by at least one of the first and second portable electronic devices, periodically generating a unique random number and wirelessly sending the unique random number to the other of the first and second portable electronic devices whenever it is determined that the first and second portable electronic devices are in close proximity to one another, and storing the received unique random number on the first or second portable electronic device respectively.

7. The method of claim 6, further comprising, by the first portable electronic device, periodically sending a request to a server to determine if a notification associated with the unique random number of the first portable electronic device is stored, wherein the notification corresponds to the first notification.

8. The method of claim 6 or 7, further comprising, if it is determined that the updated score is greater than the predetermined score, sending, to the server, the updated score and a list of the unique random numbers of all portable electronic devices that the first portable electronic device has been determined to have been in close proximity to, and, by the server, storing for each received unique random number a score based on the updated score of the first portable electronic device so that whenever a portable electronic device of all portable electronic devices that the first portable electronic device has been determined to have been in close proximity to sends a notification associated with its unique random number to the server, the server will send the updated score of the first portable electronic device.

9. The method of any of the preceding claims, wherein data including a number of times and/or the total duration the second portable device is determined to have been in close proximity to the first portable electronic device is stored in a memory of the first portable electronic device and/or the second portable electronic device.

10. The method of any of the preceding claims, wherein if it is determined that the updated score is greater than the predetermined score, an alert notification is displayed to the user on the first portable electronic device.

11. The method of any of the preceding claims, further comprising determining a location and time for each of the determinations of the portable electronic devices having been in close proximity to one another and displaying the determined location and times on a map on the first portable electronic device.

12. The method of any of the preceding claims, wherein if it is determined that the updated score is greater than the predetermined score, an alert notification is sent by the first portable electronic device to a server of an organisation managing the spread of infection.

13. A computer-implemented method (300) comprising the steps of:

receiving (302), at an electronic device at a first time (T1), a first notification that a first user of a first portable electronic device is infected;
in response to the first notification, storing one or more identifiers of at least one second user of at least one second portable electronic device determined to have been in close proximity with the first portable electronic device in a list (310);
receiving, at the electronic device at a second time (T2) after the first time, a second notification that a third user of a third portable electronic device is infected;

in response to the second notification, determining whether the third portable electronic device has been in close proximity with the first portable electronic device and the second portable electronic device after the second portable electronic device was determined to have been in close proximity with the first portable electronic device;

if it is determined (306) that the third portable electronic device has been in close proximity with the second portable electronic device but not with the first portable electronic device and it is determined (304, 306) that there are no further portable electronic devices that have been determined to have been in close proximity of the third portable electronic device, sending (308), by the electronic device, an alert notification to the second portable electronic device.

14. The method of claim 10, if it is determined that the third portable electronic device has been in close proximity with the second portable electronic device but not with the first portable electronic device and it is determined (304, 306) that there are no further portable electronic devices that have been determined to have been in close proximity of the third portable electronic device, sending (308), by the electronic device, an alert notification to one or more portable electronic devices of one or more users identified in the list which have been determined to have been in close proximity to the second portable electronic device.

15. The method of claim 10 to 12, wherein at least part of the list is stored (310) on the one or more of the portable electronic devices.

# FIG. 1

100

102

104

106

Y

N

108

110

**FIG. 2**

# FIG. 3

300

302

304

Y          N

306

Y          N

308

310

**FIG. 4**

Europäisches
Patentamt
European
Patent Office
Office européen
des brevets

**EUROPEAN SEARCH REPORT**

Application Number

EP 20 17 3770

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Apple & Google: "Exposure Notification Frequently Asked Questions Preliminary - Subject to Modification and Extension", <br><br> 4 May 2020 (2020-05-04), XP055727735, Retrieved from the Internet: URL:https://covid19-static.cdn-apple.com/applications/covid19/current/static/contact-tracing/pdf/ExposureNotification-FAQv1.0.pdf [retrieved on 2020-09-04] | 1-12,14, 15 | INV. G16H50/80 H04W4/00 G08B21/00 |
| A | * page 4 - page 7 * ----- | 13 | |
| A | Macrumors: "Apple and Google Partner on Opt-In COVID-19 Contact Tracing Technology to Be Added to iPhone and Android Smartphones - MacRumors", <br><br> 10 April 2020 (2020-04-10), XP055727908, Retrieved from the Internet: URL:https://www.macrumors.com/2020/04/10/apple-google-covid-19-contact-tracing/ [retrieved on 2020-09-07] * the whole document * ----- | 1 | |
| A | Macrumors: "UK Pursues Centralized Coronavirus Contact Tracing App, Rejects Apple-Google Solution - MacRumors", <br><br> 27 April 2020 (2020-04-27), XP055727911, Retrieved from the Internet: URL:https://www.macrumors.com/2020/04/27/uk-pursues-centralized-coronavirus-tracking-app/ [retrieved on 2020-09-07] * the whole document * ----- <br><br> -/-- | 1 | TECHNICAL FIELDS SEARCHED (IPC) <br><br> G16H H04W G08B |

The present search report has been drawn up for all claims

| Place of search | Date of completion of the search | Examiner |
|---|---|---|
| Munich | 10 September 2020 | Rivera, Pedro V. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 17 3770

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Macrumors: "Apple's Contact Tracing System Will Require Verification When Users Report Testing Positive for COVID-19 - MacRumors", , 13 April 2020 (2020-04-13), XP055727912, Retrieved from the Internet: URL:https://www.macrumors.com/2020/04/13/apple-contact-tracing-will-require-verification/ [retrieved on 2020-09-07] * the whole document * | 1 | |

TECHNICAL FIELDS
SEARCHED (IPC)

The present search report has been drawn up for all claims

| Place of search | Date of completion of the search | Examiner |
|---|---|---|
| Munich | 10 September 2020 | Rivera, Pedro V. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 2 of 2